Europäisches Patentamt

European Patent Office

Office européen des brevets

(11) Veröffentlichungsnummer: **0 443 598 B1**

(12) **EUROPÄISCHE PATENTSCHRIFT**

(45) Veröffentlichungstag der Patentschrift: **17.05.95**

(51) Int. Cl.6: **C07K 7/00**, A61K 38/04, G01N 33/86

(21) Anmeldenummer: **91102607.8**

(22) Anmeldetag: **22.02.91**

(54) **Die Blutgerinnung inhibierende Peptide, Verfahren zu ihrer Herstellung und ihre Verwendung.**

(30) Priorität: **22.02.90 DE 4005591**

(43) Veröffentlichungstag der Anmeldung:
**28.08.91 Patentblatt 91/35**

(45) Bekanntmachung des Hinweises auf die Patenterteilung:
**17.05.95 Patentblatt 95/20**

(84) Benannte Vertragsstaaten:
**AT BE CH DE DK ES FR GB GR IT LI LU NL SE**

(56) Entgegenhaltungen:
**EP-A- 0 276 014**

**THE JOURNAL OF BIOLOGICAL CHEMISTRY, Band 264, Nr. 15, 25. Mai 1989, Seiten8692-8698, The American Society for Biochemistry and Molecular Biology, Inc.,US; J.M. MARAGANORE et al.: "Anticoagulant activity of synthetic hirudinpeptides"**

(73) Patentinhaber: **BEHRINGWERKE Aktiengesellschaft
Postfach 1140
D-35001 Marburg (DE)**

(72) Erfinder: **Stüber, Werner, Dr.
Cölber Weg 12
W-3551 Lahntal (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

EP 0 443 598 B1

## Beschreibung

Die vorliegende Erfindung betrifft Peptide, welche die Blutgerinnung inhibieren, Verfahren zu ihrer Herstellung und ihre Verwendung als Anticoagulantien.

Anticoagulantien sind von hoher therapeutischer Relevanz bei der Behandlung von verschiedenen, die Blutgerinnung betreffenden Erkrankungen wie die disseminierte intravaskulare Gerinnung, der Myocardinfarkt und die tiefe Venenthrombose. Zur Therapie dieser Erkrankungen werden zur Zeit Anticoagulantien eingesetzt wie das aus Humanplasma gewonnene Antithrombin III.

In jüngerer Zeit wird ein Polypeptid aus dem Blutegel (Hirudo medicinalis) bestehend aus 65 Aminosäuren als Anticoagulans geprüft. Allerdings ist der Einsatz des Hirudins, wie dieses Peptid auch genannt wird, durch diverse Nachteile gekennzeichnet. Ein Nachteil ist die Problematik der geringen Verfügbarkeit dieser Substanz. Mögliche Schwierigkeiten können auch in dem relativ hohen Molekulargewicht dieses Peptids liegen, wodurch potentiell die Gefahr einer Antikörperbildung gegeben ist.

Diese Nachteile konnten dadurch umgangen werden, daß niedermolekulare Peptide als Antikoagulantien entwickelt wurden, die eine hohe Homologie zum C-terminalen Bereich des Hirudins aufweisen. Solche Peptide werden in der Anmeldung EP-A 0 276 014, EP 0 333 356 und in einer Publikation von J. M. Maraganore et al., J. Biol. Chem. 264, 8692-8698 (1989) beschrieben.

Wie daraus hervorgeht, ist ein Peptid der Struktur Asn-Gly-Asp-Phe-Glu-Glu-Ile-Pro-Glu-Glu-Tyr-Leu-OH von besonderem Interesse. Ganz besondere Wirksamkeit konnte bei einem Peptid nachgewiesen werden, dessen Tyrosin (Tyr) an der phenolischen Gruppe sulfatiert ist. Dies entspricht im übrigen dem nativen Hirudin, dessen Tyrosin an der Position 63 sulfatiert ist.

Von Interesse sind auch cyclische Peptide. Diese enthalten zusätzlich zur gerade erwähnten Sequenz am C- und N-Terminus die Aminosäure Cystein, die eine Cyclisierung im Sinne eines Disulfids erlaubt. Anstelle einer Disulfidbrücke können andere chemische Funktionen stehen, vorzugsweise eine Verknüpfung über eine Amidbindung.

Seiner chemischen Natur nach ist ein sulfatiertes Tyrosin ein Ester der Schwefelsäure, so daß die Bindung zwischen dem Schwefelatom und dem phenolischen Sauerstoffatom durch Hydrolyse gespalten werden kann. Solche Peptide haben den Nachteil einer verringerten antikoagulatorischen Wirksamkeit.

Nach dem Stand der Technik wird deshalb versucht, die Sulfatierung an Tyrosin durch eine nachträgliche chemische Umsetzung zu erreichen. Zu diesem Zweck werden die unsulfatierten Hirudinpeptide mit Dicyclohexylcarbodiimid und Schwefelsäure in organischen Lösemitteln zu Reaktion gebracht. Die Sulfatierung kann auch durch eine Reaktion des tyrosinhaltigen Peptids mit Schwefeltrioxid Triethylammoniumsalz in Pyridin oder Chlorsulfonsäure erreicht werden.

Diese Umsetzungen sind allerdings durch die Nachteile gekennzeichnet, daß Nebenreaktionen an Phenylalanin oder eine nicht selektive Sulfatierung bei Vorhandensein mehrerer Tyrosinreste stattfinden können. Dadurch können auch hohe Ausbeuteverluste entstehen.

Der vorliegenden Erfindung lag deshalb die Aufgabe zu Grunde, diese Nachteile zu beheben und Peptide mit überlegenen physikalischen, chemischen und physiologischen Eigenschaften bereitzustellen.

Diese Aufgabe wurde dadurch überraschenderweise gelöst, daß in den Peptiden die Aminosäure Tyrosin oder $Tyr(SO_3H)$ durch die Aminosäure $Phe(SO_3H)$ oder $Phe(PO_3H_2)$ oder $Pgl(SO_3H)$ (Pgl bedeutet Phenylglycin) oder $Pgl(PO_3H_2)$ ersetzt wurde. Die Sulfonat- oder Phosphatgruppe ist dabei vorzugsweise in der para Stelle im Phenylring des Phenylalanins gebunden, eine Bindung in der meta Position ist jedoch ebenfalls möglich.

Gegenstand der Erfindung ist daher ein Peptid der allgemeinen Formel:

A1-A2-A3-A4-A5-A6-A7-A8-A9-A10-A11-A12-A13-A14-A15

worin

A1   Wasserstoff, Cystein, eine oder zwei Alkylgruppen mit 1-4 C-Atomen, eine Acylgruppe mit 2-10 C-Atomen, eine Acylgruppe mit 2-10 C-Atomen und einer weiteren Carboxylgruppe oder eine in der Peptidchemie übliche Schutzgruppe,

A2   eine Bindung, Asn, Asp, Gln oder Glu,

A3   eine Bindung, Gly oder Ala,

A4   Glu oder Asp,

A5   Phe, Tyr, Trp, Pgl (Phenylglycin) oder Nal (Naphthylalanin),

A6   Glu oder Asp,

A7   Glu, Asp, Pro oder Ala,

A8   Ile, Leu, Val, Nle oder Phe,

A9   Pro oder Hyp,

A10   Glu oder Asp,

2

A11     Glu oder Asp,

A12     Phe($SO_3H$) oder Phe($PO_3H_2$) (vorzugsweise in p-Stellung) oder Pgl($So_3H$) oder Pgl($PO_3H_2$) (vorzugsweise in p-Stellung),

A13     eine Bindung, Leu, Ile, Val, oder Ala,

A14     eine Bindung, Gln, Asn, Glu, Asp oder Cys und

A15     Cys, Cys-amid, eine OH-Gruppe der alpha-Carboxylgruppe, frei oder mit einem niederen Alkohol mit bis zu 4 C-Atomen verestert, die auch als Carboxamidfunktion vorliegen kann, deren Wasserstoffe gegebenenfalls mit Alkylgruppen mit bis zu 4 C-Atomen ersetzt sein können,

sind.

Ist A1 die Aminosäure Cystein, so kann die Aminogruppe auch acetyliert sein.

Die Synthese der erfindungsgemäßen Peptide erfolgt nach an sich hinreichend bekannten Methoden (G. Barany und R. B. Merrifield in "The Peptides" (E. Gross und I. Meienhofer, Ed.)).

Die Aminosäuren Phe($SO_3H$) oder Pgl($SO_3H$) können durch Sulfonierung von Phenylalanin bzw. Phenylglycin, die in der D-, L-, oder D,L-, bevorzugt in der L-Form vorliegen, gewonnen werden. Als Sulfonierungsmittel kommt Schwefelsäure in Frage, die vorzugsweise noch Schwefeltrioxid enthält.

Diese Aminosulfonsäuren wurden nach C. D. Chang et al., Int. J. Peptide Protein Res. 15, 59 (1980) bekannten Verfahren an der Aminogruppe mit einer Schutzgruppe versehen, um ein erfindungsgemäßes Peptid aufbauen zu können. Als Schutzgruppen kommen dabei in Frage: Boc-,Z-,Bpoc-,Ddz- und bevorzugt die Fmoc-Gruppe.

Der Aufbau der Peptide erfolgte entweder nach einer in Lösung arbeitenden Peptidsynthesemethode unter Einbezug bekannter Arbeitsweisen (E. Wünsch in "Houben-Weyl, Synthese von Peptiden I", G. Thieme Verlag, Stuttgart (1974)) oder nach ebenfalls bekannten (s. o.).

Solid-Phase-Methoden, wobei vorzugsweise die Fmoc-Chemie benutzt wurde.

Die Sulfonat- oder Phosphatgruppe wurde ungeschützt in der Synthese eingesetzt.

Bei der Festphasenpeptidsynthese wurde die Peptidkette an quervernetztem Polystyrol (1 % Divinylbenzol) aufgebaut (später Harz genannt). Für die Synthese von Peptiden mit freien Carboxylgruppen wurden Anker auf Basis von Alkoxybenzylalkohol eingesetzt. Für Peptidamide wurden Amidanker benützt (Int. J. Peptide Protein Res. 34, 262-267, 1989), die nichtalkylierte Peptidamide produzieren. Der Einbau der einzelnen geschützten Aminosäuren erfolgte nach einem repetitiven Muster:

- Vorlage des Fmoc-Aminosäure (bzw. Amidanker)-Harzes in einem vollautomatischen Peptidsynthesizer
- Waschen des Harzes mit DMF, Dichlormethan oder N-Methylpyrrolidon (ca. 15 ml/mg)
- Abspaltung der Fmoc-Gruppe mit 20 % Piperidin in Dimethylformamid oder N-Methylpyrrolidon (vorzugsweise 1 x 3 min und 1 x 10 min)
- Auswaschung des Piperidins mit DMF, Dichlormethan, N-Methylpyrrolidon oder einem Alkohol, vorzugsweise Isopropanol
- Kopplung der Aminosäure mit einem Carbodiimid, vorzugsweise Diisopropylcarbodiimid, gegebenenfalls unter Zusatz von HOBt, HOSu oder unter Benutzung von BOP oder TBTU gegebenenfalls unter Zusatz von HOBt, vorzugsweise in DMF oder in N-Methylpyrrolidon.

Die Seitenketten der trifunktionellen Aminosäuren waren wie folgt geschützt:

- Asp und Glu als t.-Butylester
- Hyp und Tyr als t.-Butylether
- Cys als Tritylether oder tert.-Butyldisulfid.

An Stelle der oben dargestellten Fmoc-Chemie können diese Peptide auch mit der Boc-Strategie (J. M. Stewart und J. D. Young "Solid Phase Peptide Synthesis" Pierce Chemical Co., 1984, S. 71-95) aufgebaut werden, da durch den repetitiven Einsatz von Trifluoressigsäure die Sulfonatgruppe nicht geschädigt wird.

Die Peptide wurden bei der Fmoc-Chemie mittels Trifluoressigsäure, vorzugsweise unter Zusatz eines Scavengers vom Harz abgespalten und mit Ether kristallisiert. Nach Reinigung mittels reversed-phase-Chromatographie wurde ihre Zusammensetzung mittels Aminosäureanalyse und FAB-Massenspektrometrie bestätigt.

Wurden Cystein enthaltende Peptide hergestellt, s o wurde im Falle von Cys(Trt) die Tritylschutz simultan bei der Peptidabspaltung entfernt, vorausgesetzt die Abspaltungsmischung enthielt ein Thiol, vorzugsweise Ethandithiol, als Zusatz.

Wurde Cys (StBu) eingesetzt, so wurde die S-tBu Gruppe vorzugsweise nach Abspaltung des Peptids entfernt. Hierzu kamen bekannte Verfahren, wie z.B. die Behandlung mit Tri-n-Butylphosphin oder Dithiothreitol zum Einsatz. Vorzugsweise wurde die Dithiothreitol-Entschützung benutzt.

Die Cyclisierung via S-S-Brücke ließ sich durch bekannte oxidative Verfahren durchführen.

EP 0 443 598 B1

Vorzugsweise wurde das cysteinhaltige Peptid in einer Konzentration von 0,1 bis 0,001 mM in Ammoniumhydrogencarbonatpuffer (0,01 M) gelöst und mehrere Stunden an der Luft geschüttelt. Die Cyclisierung wurde mittels HPLC verfolgt.

Weitere Cyclisierungsverfahren, wie z.B. Oxidationen mit Jod, beispielsweise in Essigsäure, oder $K_3[Fe(CN)_6]$ sind hierzu ebenfalls geeignet.

Als Alternative bietet sich die Herstellung nach einem in Lösung arbeitenden Verfahren an, wobei zunächst einzelne Fragmente des gesamten Peptids hergestellt werden. Die Kondensation einzelner, geschützter Aminosäuren zu Peptidsegmenten erfolgte in Lösemittel wie DMF und Tetrahydrofuran oder Mischungen hieraus. Die Kopplung der Aminosäuren wurde mittels Carbodiimiden, wie bei der Festphasensynthese ausgeführt. Einzelne Segmente wurden dann zu den gesamten Peptiden zusammengesetzt. Nach Abspaltung der Schutzgruppen wurden die Peptide ebenfalls gereinigt und charakterisiert.

Die Peptide wurden in einem funktionellen Test auf ihre Wirksamkeit getestet.

Abkürzungen:

| | |
|---|---|
| Asn | L-Asparagin |
| Asp | L-Asparaginsäure |
| Cys | L-Cystein |
| Gln | L-Glutamin |
| Glu | L-Glutaminsäure |
| Gly | Glycin |
| Ala | L-Alanin |
| Tyr | L-Tyrosin |
| Phe | Phenylalanin |
| Trp | L-Tryptophan |
| Pgl | L-Phenylglycin |
| Pro | L-Prolin |
| Ile | L-Isoleucin |
| Leu | L-Leucin |
| Nle | Norleucin |
| Val | L-Valin |
| Nal | L-Naphthylalanin |
| Hyp | L-Hydroxyprolin |
| Boc | t.-Butyloxycarbonyl |
| Z | Benzyloxycarbonyl |
| Bpoc | Biphenylylpropyloxycarbonyl |
| Ddz | Dimethyldimethoxybenzyloxycarbonyl |
| Fmoc | Fluorenylmethyloxycarbonyl |
| DMF | Dimethylformamid |
| HOBt | 1-Hydroxysuccinimid |
| Ac | Acetyl |
| Suc | Succinimidyl |
| BOP | Benzotriazol-1-yl-oxy-tris(dimethylamino) phosphonium hexafluorophosphat |
| TBTU | 2(1H-Benzotriazol-1-yl)-1,1,3,3-tetramethyluronium tetrafluorophosphat |
| Osn | Succinimidester |
| TFA | Trifluoressigsäure |
| DIC | Diisopropylcarbodiimid |
| S-tBu | Tert.-Butylthio |
| Trt | Trityl |
| FAB | Fast Atom Bombardment |

4

**Beispiele**

**Beispiel 1:**

Herstellung von Fmoc-Phe(SO$_3$H)

20 Gramm L-Phenylalanin wurden in einer Mischung aus 17 ml 30 %igem Oleum und 20 ml conc. Schwefelsäure portionsweise gelöst. Der Ansatz wurde 1 Stunde auf 100°C erhitzt und dann in 200 ml Eiswasser gegossen. Die Säure wurde mit Bariumhydroxid neutralisiert und das Bariumsulfat abfiltriert. Das Filtrat wurde über eine Säule (Dowex 50WX2, 50-100 mesh, Dimension 230 x 32 mm) mit Wasser als Eluens chromatographiert. Nach Abdampfen des Lösemittels wurden 18.5 Gramm L-para-Sulfophenylalanin erhalten.

7.35 Gramm der Aminosäure wurden in 150 ml 10 %iger Natriumcarbonatlösung aufgenommen. Dazu wurde eine Lösung von 10 Gramm Fmoc-OSu in 300 ml Dioxan unter Rühren zugesetzt. Der Ansatz wurde alsbald gallertartig und wurde 2 Stunden bei Raumtemperatur gerührt. Der Niederschlag wurde abfiltriert und das Dioxan abgedampft. Die wässrige Lösung wurde 3 x mit Ether extrahiert und mit 1N Salzsäure auf pH2 angesäuert.

Eine weitere Verunreinigung wurde mit Ethylacetat extrahiert. Die Wasserphase wurde mittels eines Rotationsverdampfers eingedampft und der kristalline Rückstand wurde über [R]Sicapent im Vakuum getrocknet.

**Beispiel 2:**

Asn-Gly-Asp-Phe-Glu-Glu-Ile-Pro-Glu-Glu-Phe(SO$_3$H)-Leu-OH

0.83 Gramm Fmoc-Leu-Harz (o.5 mMol) wurden an einem Peptidsynthesizer der Firma Advanced Chemtech (Louisville, Kentucky, USA) nach Herstellerangaben zur Kopplung der nächsten Aminosäure vorbereitet. Fmoc-Phe(SO$_3$H) (1.5 mMol) und 2.25 mMol HOBt wurden in 15 ml DMF und 15 ml DMSO gelöst und mit 1.6 mMol DIC versetzt. Nach einer Stunde wurde der Ansatz zum Leu-Harz gegeben und zwei Stunden gekoppelt. Die Synthese wurde dann nach Standardverfahren fertiggestellt. Zur Kopplung wurde TBTU mit 3-fachem Aminosäureüberschuß benutzt. Die Kopplungszeit war je 35 Minuten. Das Peptidharz wurde mit 27 ml TFA, 1,5 ml Ethandithiol und 1 g Resorcin eine Stunde lang behandelt. Die Peptidlösung wurde in Ether kristallisiert, abfiltriert und getrocknet. Rohausbeute 405 mg. 110 mg dieses Rohpeptids wurden an einer HPLC-Säule (Shandon, RP-18, 250 x 20 mm) mit einem Gradienten aus 0.1 TFA/Acetonitril gereinigt. Das Peptid wurde durch Lyophilisation gewonnen (Ausbeute 48 mg). Nach Hydrolyse wurde der Peptidgehalt auf 78 % bestimmt.

Die Aminosäurezusammensetzung ist dem beiliegenden Diagramm zu entnehmen und entsprach dem Erwartungswert.

| Aminosäureanalyse: | Phe(SO$_3$H) | 0.95 (1) |
|---|---|---|
| | Asp | 1.94 (2) |
| | Glu | 4.18 (4) |
| | Pro | 1.10 (1) |
| | Gly | 1.00 (1) |
| | Ile | 0.90 (1) |
| | Leu | 0.90 (1) |
| | Phe | 0.99 (1) |

Austestung:

10 mg des Peptids wurden in 1 ml Puffer (20 mM Tris, 150 mM NaCl pH 7.5) gelöst. Das Peptid wurde in der partiellen Thromboplastinzeit (PTT) gegenüber einem Peptid mit derselben Sequenz getestet, das anstelle von Phe(SO$_3$ H) jedoch Tyr enthielt.

EP 0 443 598 B1

PTT-Test:

100 Mikroliter Standard Humanplasma
100 Mikroliter Puffer (s.o.)
100 Mikroliter Kaolin/Pathromtin Reagenz (BEHRINGWERKE AG)
2 Minuten bei 37 ° C
100 Mikroliter $CaCl_2$-Lösung

Ergebnis:

| Verdünnung | Gerinnungszeiten in Sekunden | |
| --- | --- | --- |
| | Peptid lt. Beispiel | Peptid mit Tyr |
| 1:4 | 147.2 | 105.2 |
| 1:8 | 111.8 | 80.5 |
| 1:16 | 89.2 | 71.0 |
| 1:32 | 81.5 | 65.0 |
| 1:64 | 72.8 | 57.7 |
| 1:128 | 63.0 | 52.8 |
| 1:256 | 56.3 | 47.3 |
| 1:512 | 52.2 | 44.7 |
| 1:1024 | 46.8 | 41.7 |

Leerwert (ohne Peptid) 38.8

Die jeweiligen Aminosäure-Analysen und FAB-Massenspektren stimmten mit den zu erwartenden Ergebnissen überein.

6

Entsprechend werden die folgenden Peptide hergestellt:

H-Asn-Gly-Asp-Phe-Glu-Glu-Ile-Pro-Glu-Glu-Phe(SO$_3$H)-Leu-OH

H-Gly-Asp-Phe-Glu-Glu-Ile-Pro-Glu-Glu-Phe(SO$_3$H)-Leu-Gln-OH

Ac-Asp-Phe-Glu-Glu-Ile-Pro-Glu-Glu-Phe(SO$_3$H)-Leu-OH

Sar-Glu-Tyr-Glu-Glu-Ile-Pro-Glu-Glu-Phe(SO$_3$H)-Ile-OH

Ac-Asn-Ala-Asp-Pgl-Glu-Glu-Ile-Pro-Glu-Glu-Phe(SO$_3$H)-Leu-OH

H-Asp-Trp-Glu-Glu-Ile-Pro-Glu-Glu-Phe(SO$_3$H)-Leu-Gln-OH

H-Asn-Gly-Asp-Pgl-Glu-Glu-Ile-Pro-Glu-Glu-Phe(SO$_3$H)-Leu-OH

H-Asp-Asp-Phe-Glu-Glu-Ile-Pro-Glu-Glu-Phe(SO$_3$H)-Asp-OH

Suc-Asp-Phe-Glu-Glu-Ile-Pro-Glu-Glu-Phe(SO$_3$H)-Leu-OH

Suc-Asp-Pgl-Glu-Glu-Ile-Pro-Glu-Glu-Phe(SO$_3$H)-Leu-OH

Ac-Gly-Asp-Phe-Glu-Glu-Nle-Pro-Glu-Glu-Phe(SO$_3$H)-Ile-Asn-NH$_2$

Ac-Gly-Asp-Tyr-Glu-Glu-Val-Pro-Glu-Glu-Phe(SO$_3$H)-Leu-NH$_2$

Suc-Glu-Ala-Asp-Tyr-Glu-Pro-Leu-Pro-Glu-Glu-Phe(SO$_3$H)-Leu-OH

Ac-Gln-Ala-Asp-Phe-Asp-Asp-Phe-Asp-Asp-Phe(SO$_3$H)-Ala-NH$_2$

Ac-Gly-Asp-Phe-Glu-Glu-Ile-Pro-Glu-Glu-Phe(SO$_3$H)-D-Leu-OH

Ac-Gly-Asp-Phe-Glu-Glu-Ile-Pro-Glu-Glu-D-Phe(SO$_3$H)-Leu-Gln-OH

H-Asn-Gly-Asp-Phe-Glu-Glu-Ile-Pro-Glu-Glu-Pgl(SO$_3$H)-Leu-OH

Ac-Asp-Pgl-Glu-Glu-Ile-Pro-Glu-Glu-Pgl(SO$_3$H)-Leu-OH

Ac-Asp-Nal-Glu-Glu-Ile-Pro-Glu-Glu-Phe(SO$_3$H)-Leu-OH

Cys-Asn-Gly-Asp-Phe-Glu-Glu-Ile-Pro-Glu-Glu-Phe(SO$_3$H)-Leu-Cys-OH

Cys-Asn-Gly-Asp-Phe-Glu-Glu-Ile-Pro-Glu-Glu-Phe(SO$_3$H)-Leu-Gln-Cys-OH

Cys-Asn-Gly-Asp-Tyr-Glu-Glu-Ile-Pro-Glu-Glu-Phe(SO$_3$H)-Leu-Cys-OH

EP 0 443 598 B1

**Patentansprüche**
**Patentansprüche für folgende Vertragsstaaten : AT, BE, CH, DE, DK, FR, GB, IT, LI, LU, NL, SE**

1. Peptide der Formel I

   A1-A2-A3-A4-A5-A6-A7-A8-A9-A10-A11-A12-A13-A14-A15

   worin

   A1  Wasserstoff, Cystein, Acetylcystein, eine oder zwei Alkylgruppen mit 1-4 C-Atomen, eine Acylgruppe mit 2-10 C-Atomen, eine Acylgruppe mit 2-10 C-Atomen und einer weiteren Carboxylgruppe oder eine in der Peptidchemie übliche Schutzgruppe ist,

   A2  eine Bindung, Asn, Asp, Gln oder Glu,

   A3  eine Bindung, Gly oder Ala,

   A4  Glu oder Asp,

   A5  Phe, Tyr, Trp, Pgl (Phenylglycin) oder Nal (Naphthylalanin),

   A6  Glu oder Asp,

   A7  Glu, Asp, Pro oder Ala,

   A8  Ile, Leu, Val, Nle oder Phe,

   A9  Pro oder Hyp,

   A10  Glu oder Asp,

   A11  Glu oder Asp,

   A12  Phe($SO_3H$) oder Phe($PO_3H_2$) (vorzugsweise in p-Stellung) oder Pgl($SO_3H$) oder Pgl($PO_3H_2$) (vorzugsweise in p-Stellung),

   A13  eine Bindung, Leu, Ile, Val, oder Ala,

   A14  eine Bindung, Gln, Asn, Glu, Asp oder Cys und

   A15  Cys, Cys-amid, eine OH-Gruppe der alpha-Carboxylgruppe, frei oder mit einem niederen Alkohol mit bis zu 4 C-Atomen verestert, die auch als Carboxamidfunktion vorliegen kann, deren Wasserstoffe gegebenenfalls mit Alkylgruppen mit bis zu 4 C-Atomen ersetzt sein können,

   sind.

2. Peptid nach Anspruch 1, in dem A12 Sulfophenylalanin in der D- oder L- Form ist.

3. Peptid nach Anspruch 1, in dem A12 Sulfophenylglycin in der D- oder L- Form ist.

4. Peptid nach Anspruch 1, in dem A1 Wasserstoff, Methyl, Acetyl, Benzoyl oder Succinyl ist.

5. Peptid nach Anspruch 1 mit der Struktur H-Asn-Gly-Asp-Phe-Glu-Glu-Pro-Glu-Glu-Phe($SO_3H$)-Leu-OH.

6.   Peptid nach Anspruch 1 mit der Struktur

H-Gly-Asp-Phe-Glu-Glu-Ile-Pro-Glu-Glu-Phe(SO$_3$H)-Leu-Gln-OH,

Ac-Asp-Phe-Glu-Glu-Ile-Pro-Glu-Glu-Phe(SO$_3$H)-Leu-OH,

Sar-Glu-Tyr-Glu-Glu-Ile-Pro-Glu-Glu-Phe(SO$_3$H)-Ile-OH,

Ac-Asn-Ala-Asp-Pgl-Glu-Glu-Ile-Pro-Glu-Glu-Phe(SO$_3$H)-Leu-OH,

H-Asp-Trp-Glu-Glu-Ile-Pro-Glu-Glu-Phe(SO$_3$H)-Leu-Gln-OH,

H-Asn-Gly-Asp-Pgl-Glu-Glu-Ile-Pro-Glu-Glu-Phe(SO$_3$H)-Leu-OH,

H-Asp-Asp-Phe-Glu-Glu-Ile-Pro-Glu-Glu-Phe(SO$_3$H)-Asp-OH,

Suc-Asp-Phe-Glu-Glu-Ile-Pro-Glu-Glu-Phe(SO$_3$H)-Leu-OH,

Suc-Asp-Pgl-Glu-Glu-Ile-Pro-Glu-Glu-Phe(SO$_3$H)-Leu-OH,

Ac-Gly-Asp-Phe-Glu-Glu-Nle-Pro-Glu-Glu-Phe(SO$_3$H)-Ile-Asn-NH$_2$,

Ac-Gly-Asp-Tyr-Glu-Glu-Val-Pro-Glu-Glu-Phe(SO$_3$H)-LeuNH$_2$,

Suc-Glu-Ala-Asp-Tyr-Glu-Pro-Leu-Pro-Glu-Glu-Phe(SO$_3$H)-Leu-OH,

Ac-Gln-Ala-Asp-Phe-Asp-Asp-Phe-Asp-Asp-Phe(SO$_3$H)-Ala-NH$_2$,

```
Ac-Gly-Asp-Phe-Glu-Glu-Ile-Pro-Glu-Glu-Phe(SO₃H)-
D-Leu-OH,


Ac-Gly-Asp-Phe-Glu-Glu-Ile-Pro-Glu-Glu-D-Phe(SO₃H)-
Leu-Gln-OH,


H-Asn-Gly-Asp-Phe-Glu-Glu-Ile-Pro-Glu-Glu-Pgl(SO₃H)-
Leu-OH,


Ac-Asp-Pgl-Glu-Glu-Ile-Pro-Glu-Glu-Pgl(SO₃H)-Leu-OH
oder


Ac-Asp-Nal-Glu-Glu-Ile-Pro-Glu-Glu-Phe(SO₃H)-Leu-OH.
```

7. Verfahren zur Herstellung eines Peptids nach Anspruch 1 mittels Festphasenpeptidsynthese oder in Lösung arbeitender Synthese.

8. Verfahren zur Herstellung eines Peptids nach Anspruch 1, dadurch gekennzeichnet, daß die Peptidkette an einem polymeren Träger mittels repetitiver Kopplung geschützter Aminosäuren oder Oligopeptiden aufgebaut wird und davon abgespalten wird.

9. Verfahren zur Herstellung eines Peptids nach Anspruch 1, dadurch gekennzeichnet, daß die Peptidkette in Lösung unter Benutzung geschützter Aminosäuren oder geschützter Oligopeptide zusammengebaut wird und das Peptid durch Abspaltung der Schutzgruppen gewonnen wird.

10. Verfahren zur Herstellung eines Peptids der Formel I, dadurch gekennzeichnet, daß geschützte Aminosäurederivate oder Peptidsegmente in Lösung oder an einer Festphase aneinandergekoppelt werden und durch Abspaltung der Schutzgruppen sowie im Falle einer Festphase durch Abspaltung vom Trägerharz erhalten werden, wobei im Falle von Cystein enthaltenden Peptiden ein oxidativer Ringschluß erfolgen kann.

11. Diagnostisches oder therapeutisches Mittel enthaltend eine Verbindung nach Anspruch 1.

**Patentansprüche für folgende Vertragsstaaten : ES, GR**

1. Verfahren zur Herstellung eines Peptids der Formel I
A1-A2-A3-A4-A5-A6-A7-A8-A9-A10-A11-A12-A13-A14-A15
worin
A1     Wasserstoff, Cystein, Acetylcystein, eine oder zwei Alkylgruppen mit 1-4 C-Atomen, eine Acylgruppe mit 2-10 C-Atomen, eine Acylgruppe mit 2-10 C-Atomen und einer weiteren Carboxylgruppe oder eine in der Peptidchemie übliche Schutzgruppe ist,
A2     eine Bindung, Asn, Asp, Gln oder Glu,
A3     eine Bindung, Gly oder Ala,
A4     Glu oder Asp,
A5     Phe, Tyr, Trp, Pgl (Phenylglycin) oder Nal (Naphthylalanin),
A6     Glu oder Asp,
A7     Glu, Asp, Pro oder Ala,
A8     Ile, Leu, Val, Nle oder Phe,
A9     Pro oder Hyp,

A10     Glu oder Asp,

A11     Glu oder Asp,

A12     Phe($SO_3H$) oder Phe($PO_3H_2$) (vorzugsweise in p-Stellung) oder Pgl($SO_3H$) oder Phe($PO_3H_2$) (vorzugsweise in p-Stellung),

A13     eine Bindung Leu, Ile, Val, oder Ala,

A14     eine Bindung, Gln, Asn, Glu, Asp oder Cys und

A15     Cys, Cys-amid, eine OH-Gruppe der alpha-Carboxylgruppe, frei oder mit einem niederen Alkohol mit bis zu 4 C-Atomen verestert, die auch als Carboxamidfunktion vorliegen kann, deren Wasserstoffe gegebenenfalls mit Alkylgruppen mit bis zu 4 C-Atomen ersetzt sein können,

sind,

mittels Festphasenpeptidsynthese oder in Lösung arbeitender Synthese.

2.    Verfahren nach Anspruch 1, dadurch gekennzeichnet, daß die Peptidkette an einem polymeren Träger mittels repetitiver Kopplung geschützter Aminosäuren oder Oligopeptiden aufgebaut wird und davon abgespalten wird.

3.    Verfahren nach Anspruch 1, dadurch gekennzeichnet, daß die Peptidkette in Lösung unter Benutzung geschützter Aminosäuren oder geschützter Oligopeptide zusammengebaut wird und das Peptid durch Abspaltung der Schutzgruppen gewonnen wird.

4.    Verfahren nach Anspruch 1, dadurch gekennzeichnet, daß geschützte Aminosäurederivate oder Peptidsegmente in Lösung oder an einer Festphase aneinandergekoppelt werden und durch Abspaltung der Schutzgruppen sowie im Falle einer Festphase durch Abspaltung vom Trägerharz erhalten werden, wobei im Falle von Cystein enthaltenden Peptiden ein oxidativer Ringschluß erfolgen kann.

5.    Verfahren nach einem der Ansprüche 1-4, dadurch gekennzeichnet, daß ein Peptid hergestellt wird, in dem A12 Sulphonylphenylalanin in der D- oder L-Form ist.

6.    Verfahren nach einem der Ansprüche 1-4, dadurch gekennzeichnet, daß ein Peptid hergestellt wird, in dem A12 Sulphonylphenylglycin in der D- oder L-Form ist.

7.    Verfahren nach einem der Ansprüche 1-4, dadurch gekennzeichnet, daß ein Peptid hergestellt wird, in dem A1 Wasserstoff, Methyl, Acetyl, Benzoyl oder Succinyl ist.

8.    Verfahren nach einem der Ansprüche 1-4, dadurch gekennzeichnet, daß ein Peptid mit der Struktur H-Asn-Gly-Asp-Phe-Glu-Glu-Pro-Glu-Glu-Phe(SO3H)-Leu-OH hergestellt wird.

9.  Verfahren nach einem der Ansprüche 1-4, dadurch gekennzeichnet, daß ein Peptid mit der Struktur

```
H-Asn-Gly-Asp-Phe-Glu-Glu-Ile-Pro-Glu-Glu-Phe(SO3-
H)-Leu-OH,

H-Gly-Asp-Phe-Glu-Glu-Ile-Pro-Glu-Glu-Phe(SO3H)-L-
eu-Gln-OH,
Ac-Asp-Phe-Glu-Glu-Ile-Pro-Glu-Glu-Phe(SO3H)-Leu--
OH,
Sar-Glu-Tyr-Glu-Glu-Ile-Pro-Glu-Glu-Phe(SO3H)-Ile-
-OH,
Ac-Asn-Ala-Asp-Pgl-Glu-Glu-Ile-Pro-
Glu-Glu-Phe(SO3H)-Leu-OH,
H-Asp-Trp-Glu-Glu-Ile-Pro-Glu-Glu-Phe(SO3H)-Leu-
Gln-OH,
H-Asn-Gly-Asp-Pgl-Glu-Glu-Ile-Pro-Glu-Glu-Phe(SO3-
H)-Leu-OH,
H-Asp-Asp-Phe-Glu-Glu-Ile-Pro-Glu-Glu-Phe(SO3H)-
Asp-OH,
Suc-Asp-Phe-Glu-Glu-Ile-Pro-Glu-Glu-Phe-
(SO3H)-Leu-OH,
Suc-Asp-Pgl-Glu-Glu-Ile-Pro-Glu-Glu-Phe(SO3H)-Leu-
OH,


Ac-Gly-Asp-Phe-Glu-Glu-Nle-Pro-Glu-Glu-Phe(SO3H)-
Ile-Asn-NH2,
Ac-Gly-Asp-Tyr-Glu-Glu-Val-Pro-Glu-Glu-Phe(SO3H)-
LeuNH2,
Suc-Glu-Ala-Asp-Tyr-Glu-Pro-Leu-Pro-Glu-Glu-
Phe(SO3H)-Leu-OH,
Ac-Gln-Ala-Asp-Phe-Asp-Asp-Phe-Asp-Asp-Phe(SO3H)--
Ala-NH2,

Ac-Gly-Asp-Phe-Glu-Glu-Ile-Pro-Glu-Glu-Phe(SO3H)-
D-Leu-OH,
Ac-Gly-Asp-Phe-Glu-Glu-Ile-Pro-Glu-Glu-D-Phe(SO3H-
)-Leu-Gln-OH,
H-Asn-Gly-Asp-Phe-Glu-Glu-Ile-Pro-Glu-Glu-Pgl(SO3-
H)-Leu-OH,
Ac-Asp-Pgl-Glu-Glu-Ile-Pro-Glu-Glu-Pgl(SO3H)-Leu--
OH
oder
Ac-Asp-Nal-Glu-Glu-Ile-Pro-Glu-Glu-Phe(SO3H)-Leu--
OH
```

hergestellt wird.

**Claims**
**Claims for the following Contracting States : AT, BE, CH, DE, DK, FR, GB, IT, LI, LU, NL, SE**

1.  A peptide of the formula I
    A1-A2-A3-A4-A5-A6-A7-A8-A9-A10-A11-A12-A13-A14-A15
        in which
        A1     is hydrogen, cysteine, acetylcysteine, one or two alkyl groups with 1-4 carbon atoms, an
               acyl group with 2-10 carbon atoms, an acyl group with 2-10 carbon atoms and another carboxyl group, or a protective group customary in peptide chemistry,

A2     is a bond, Asn, Asp, Gln or Glu,
A3     is a bond, Gly or Ala,
A4     is Glu or Asp,
A5     is Phe, Tyr, Trp, Pgl (phenylglycine) or Nal (naphthylalanine),
A6     is Glu or Asp,
A7     is Glu, Asp, Pro or Ala,
A8     is Ile, Leu, Val, Nle or Phe,
A9     is Pro or Hyp,
A10     is Glu or Asp,
A11     is Glu or Asp,
A12     is $Phe(SO_3H)$ or $Phe(PO_3H_2)$ (preferably in the p position) or
           $Pgl(SO_3H)$ or $Pgl(PO_3H_2)$ (preferably in the p position),
A13     is a bond, Leu, Ile, Val, or Ala,
A14     is a bond, Gln, Asn, Glu, Asp or Cys and
A15     is Cys, Cys amide, an OH group of the alpha-carboxyl group, free or esterified with a lower alcohol with up to 4 carbon atoms, which can also be in the form of a carboxamide group whose hydrogens can optionally be replaced by alkyl groups with up to 4 carbon atoms.

2. A peptide as claimed in claim 1, in which A12 is sulfophenylalanine in the D or L form.

3. A peptide as claimed in claim 1, in which A12 is sulfophenylglycine in the D or L form.

4. A peptide as claimed in claim 1, in which A1 is hydrogen, methyl, acetyl, benzoyl or succinyl.

5. A peptide as claimed in claim 1 with the structure H-Asn-Gly-Asp-Phe-Glu-Glu-Pro-Glu-Glu-Phe-$(SO_3H)$-Leu-OH.

6. A peptide as claimed in claim 1 with the structure

```
H-Asn-Gly-Asp-Phe-Glu-Glu-Ile-Pro-Glu-Glu-Phe(SO3H)-
Leu-OH,
```

EP 0 443 598 B1

H-Gly-Asp-Phe-Glu-Glu-Ile-Pro-Glu-Glu-Phe($SO_3H$)-Leu-Gln-OH,

Ac-Asp-Phe-Glu-Glu-Ile-Pro-Glu-Glu-Phe($SO_3H$)-Leu-OH,

Sar-Glu-Tyr-Glu-Glu-Ile-Pro-Glu-Glu-Phe($SO_3H$)-Ile-OH,

Ac-Asn-Ala-Asp-Pgl-Glu-Glu-Ile-Pro-Glu-Glu-Phe($SO_3H$)-Leu-OH,

H-Asp-Trp-Glu-Glu-Ile-Pro-Glu-Glu-Phe($SO_3H$)-Leu-Gln-OH,

H-Asn-Gly-Asp-Pgl-Glu-Glu-Ile-Pro-Glu-Glu-Phe($SO_3H$)-Leu-OH,

H-Asp-Asp-Phe-Glu-Glu-Ile-Pro-Glu-Glu-Phe($SO_3H$)-Asp-OH,

Suc-Asp-Phe-Glu-Glu-Ile-Pro-Glu-Glu-Phe($SO_3H$)-Leu-OH,

Suc-Asp-Pgl-Glu-Glu-Ile-Pro-Glu-Glu-Phe($SO_3H$)-Leu-OH,

Ac-Gly-Asp-Phe-Glu-Glu-Nle-Pro-Glu-Glu-Phe($SO_3H$)-Ile-Asn-$NH_2$,

Ac-Gly-Asp-Tyr-Glu-Glu-Val-Pro-Glu-Glu-Phe($SO_3H$)-LeuNH_2,

Suc-Glu-Ala-Asp-Tyr-Glu-Pro-Leu-Pro-Glu-Glu-Phe($SO_3H$)-Leu-OH,

Ac-Gln-Ala-Asp-Phe-Asp-Asp-Phe-Asp-Asp-Phe($SO_3H$)-Ala-$NH_2$,

Ac-Gly-Asp-Phe-Glu-Glu-Ile-Pro-Glu-Glu-Phe($SO_3H$)-D-

14

`Leu-OH,`

`Ac-Gly-Asp-Phe-Glu-Glu-Ile-Pro-Glu-Glu-D-Phe(SO`$_3$`H)-`
`Leu-Gln-OH,`

`H-Asn-Gly-Asp-Phe-Glu-Glu-Ile-Pro-Glu-Glu-Pgl(SO`$_3$`H)-`
`Leu-OH,`

`Ac-Asp-Pgl-Glu-Glu-Ile-Pro-Glu-Glu-Pgl(SO`$_3$`H)-Leu-OH,`

`or`

`Ac-Asp-Nal-Glu-Glu-Ile-Pro-Glu-Glu-Phe(SO`$_3$`H)-Leu-OH.`

7.  A process for preparing a peptide as claimed in claim 1 by solid-phase peptide synthesis or synthesis operating in solution.

8.  A process for preparing a peptide as claimed in claim 1, which comprises the peptide chain being constructed on a polymeric support by means of repetitive coupling of protected amino acids or oligopeptides and being cleaved off therefrom.

9.  A process for preparing a peptide as claimed in claim 1, which comprises constructing the peptide chain in solution using protected amino acids or protected oligopeptides, and obtaining the peptide by eliminating the protective groups.

10. A process for preparing a peptide of the formula I, which comprises protected amino acid derivatives or peptide segments being coupled together in solution or on a solid phase and obtained by elimination of the protective groups and, in the case of a solid phase, by cleavage off the support resin, it being possible to carry out oxidative ring closure in the case of cysteine-containing peptides.

11. A diagnostic or therapeutic composition containing a compound as claimed in claim 1.

**Claims for the following Contracting States : ES, GR**

1.  A process for preparing a peptide of the formula I
    A1-A2-A3-A4-A5-A6-A7-A8-A9-A10-A11-A12-A13-A14-A15
    in which

    A1      is hydrogen, cysteine, acetylcysteine, one or two alkyl groups with 1-4 carbon atoms, an acyl group with 2-10 carbon atoms, an acyl group with 2-10 carbon atoms and another carboxyl group, or a protective group customary in peptide chemistry,

    A2      is a bond, Asn, Asp, Gln or Glu,

    A3      is a bond, Gly or Ala,

    A4      is Glu or Asp,

    A5      is Phe, Tyr, Trp, Pgl (phenylglycine) or Nal (naphthylalanine),

    A6      is Glu or Asp,

    A7      is Glu, Asp, Pro or Ala,

    A8      is Ile, Leu, Val, Nle or Phe,

    A9      is Pro or Hyp,

    A10     is Glu or Asp,

    A11     is Glu or Asp,

    A12     is Phe(SO$_3$H) or Phe(PO$_3$H$_2$) (preferably in the p position) or

EP 0 443 598 B1

Pgl(SO$_3$H) or Pgl(PO$_3$H$_2$) (preferably in the p position),

A13     is a bond, Leu, Ile, Val, or Ala,

A14     is a bond, Gln, Ash, Glu, Asp or Cys and

A15     is Cys, Cys amide, an OH group of the alpha-carboxyl group, free or esterified with a lower alcohol with up to 4 carbon atoms, which can also be in the form of a carboxamide group whose hydrogens can optionaliy be replaced by alkyl groups with up to 4 carbon atoms,

by solid-phase peptide synthesis or synthesis operating in solution.

2. The process as claimed in claim 1, which comprises the peptide chain being constructed on a polymeric support by means of repetitive coupling of protected amino acids or oligopeptides and being cleaved off therefrom.

3. The process as claimed in claim 1, which comprises the peptide chain being constructed in solution using protected amino acids or protected oligopeptides, and the peptide being obtained by eliminating the protective groups.

4. The process as claimed in claim 1, which comprises protected amino acid derivatives or peptide segments being coupled together in solution or on a solid phase and being obtained by eliminating the protective groups and, in the case of a solid phase, by cleavage off the support resin, it being possible to carry out oxidative ring closure in the case of cysteine-containing peptides.

5. The process as claimed in one of claims 1-4, which comprises a peptide being prepared in which A12 is sulfophenylalanine in the D or L form.

6. The process as claimed in one of claims 1-4, which comprises a peptide being prepared in which A12 is sulfophenylglycine in the D or L form.

7. The process as claimed in one of claims 1-4, which comprises a peptide being prepared in which A1 is hydrogen, methyl, acetyl, benzoyl or succinyl.

8. The process as claimed in one of claims 1-4, which comprises a peptide with the structure H-Asn-Gly-Asp-Phe-Glu-Glu-Pro-Glu-Glu-Phe(SO$_3$H)-Leu-OH being prepared.

16

9. The process as claimed in one of claims 1-4, which comprises a peptide with the structure H-Asn-Gly-Asp-Phe-Glu-Glu-Ile-Pro-Glu-Glu-Phe(SO$_3$H)-Leu-OH,

H-Gly-Asp-Phe-Glu-Glu-Ile-Pro-Glu-Glu-Phe(SO$_3$H)-Leu-Gln-OH,

Ac-Asp-Phe-Glu-Glu-Ile-Pro-Glu-Glu-Phe(SO$_3$H)-Leu-OH,

Sar-Glu-Tyr-Glu-Glu-Ile-Pro-Glu-Glu-Phe(SO$_3$H)-Ile-OH,

Ac-Asn-Ala-Asp-Pgl-Glu-Glu-Ile-Pro-Glu-Glu-Phe(SO$_3$H)-Leu-OH,

H-Asp-Trp-Glu-Glu-Ile-Pro-Glu-Glu-Phe(SO$_3$H)-Leu-Gln-OH,

H-Asn-Gly-Asp-Pgl-Glu-Glu-Ile-Pro-Glu-Glu-Phe(SO$_3$H)-Leu-OH,

H-Asp-Asp-Phe-Glu-Glu-Ile-Pro-Glu-Glu-Phe(SO$_3$H)-Asp-OH,

Suc-Asp-Phe-Glu-Glu-Ile-Pro-Glu-Glu-Phe(SO$_3$H)-Leu-OH,

Suc-Asp-Pgl-Glu-Glu-Ile-Pro-Glu-Glu-Phe(SO$_3$H)-Leu-OH,

Ac-Gly-Asp-Phe-Glu-Glu-Nle-Pro-Glu-Glu-Phe(SO$_3$H)-Ile-Asn-NH$_2$,

Ac-Gly-Asp-Tyr-Glu-Glu-Val-Pro-Glu-Glu-Phe(SO$_3$H)-LeuNH$_2$,

Suc-Glu-Ala-Asp-Tyr-Glu-Pro-Leu-Pro-Glu-Glu-Phe(SO$_3$H)-Leu-OH,

EP 0 443 598 B1

```
Ac-Gln-Ala-Asp-Phe-Asp-Asp-Phe-Asp-Asp-Phe(SO3H)-
Ala-NH2,

Ac-Gly-Asp-Phe-Glu-Glu-Ile-Pro-Glu-Glu-Phe(SO3H)-D-
Leu-OH,

Ac-Gly-Asp-Phe-Glu-Glu-Ile-Pro-Glu-Glu-D-Phe(SO3H)-
Leu-Gln-OH,

H-Asn-Gly-Asp-Phe-Glu-Glu-Ile-Pro-Glu-Glu-Pgl(SO3H)-
Leu-OH,

Ac-Asp-Pgl-Glu-Glu-Ile-Pro-Glu-Glu-Pgl(SO3H)-Leu-OH,

or

Ac-Asp-Nal-Glu-Glu-Ile-Pro-Glu-Glu-Phe(SO3H)-Leu-OH
```

being prepared.

**Revendications**
**Revendications pour les Etats contractants suivants : AT, BE, CH, DE, DK, FR, GB, IT, LI, LU, NL, SE**

1. Peptides de formule I

    A1-A2-A3-A4-A5-A6-A7-A8-A9-A10-A11-A12-A13-A14-A15

    dans laquelle

    A1     est un atome d'hydrogène ou un reste cystéine, acétyl-cystéine, un ou deux groupes alkyle ayant de 1 à 4 atomes de carbone, un groupe acyle ayant de 2 à 10 atomes de carbone et un autre groupe carboxy, ou l'un des groupes protecteurs usuels dans la chimie des peptides,

    A2     est une liaison, Asn, Asp, Gln ou Glu,

    A3     est une liaison, Gly ou Ala,

    A4     est Glu ou Asp,

    A5     est Phe, Tyr, Trp, Pgl (phénylglycine) ou Nal (naphtylalanine),

    A6     est Glu ou Asp,

    A7     est Glu, Asp, Pro ou Ala,

    A8     est Ile, Leu, Val, Nle ou Phe,

    A9     est Pro ou Hyp,

    A10    est Glu ou Asp,

    A11    est Glu ou Asp,

    A12    est $Phe(SO_3H)$ ou $Phe(PO_3H_2)$ (de préférence en position para) ou $Pgl(SO_3H)$ ou $Pgl(PO_3H_2)$ (de préférence en position para),

    A13    est une liaison, Leu, Ile, Val ou Ala,

    A14    est une liaison, Gln, Asn, Glu, Asp ou Cys et

    A15    est Cys, Cys-amide, un groupe OH du groupe $\alpha$-carboxy, libre ou estérifié par un alcool inférieur ayant jusqu'à 4 atomes de carbone, qui peut également se trouver sous forme de fonction carboxamide, dont les atomes d'hydrogène peuvent éventuellement être remplacés par des groupes alkyle ayant jusqu'à 4 atomes de carbone.

2. Peptide selon la revendication 1, dans lequel A12 est un reste sulfophénylalanine sous la forme D ou L.

3. Peptide selon la revendication 1, dans lequel A12 est un reste sulfophénylglycine sous la forme D ou L.

4. Peptide selon la revendication 1, dans lequel A1 est un atome d'hydrogène ou le groupe méthyle, acétyle, benzoyle ou succinyle.

18

**5.** Peptide selon la revendication 1, ayant la structure
H-Asn-Gly-Asp-Phe-Glu-Glu-Pro-Glu-Glu-Phe(SO₃H)-Leu-OH.

**6.** Peptide selon la revendication 1, ayant la structure

H-Asn-Gly-Asp-Phe-Glu-Glu-Ile-Pro-Glu-Glu-Phe(SO₃H)-Leu-OH,

H-Gly-Asp-Phe-Glu-Glu-Ile-Pro-Glu-Glu-Phe(SO₃H)-Leu-Gln-OH,

Ac-Asp-Phe-Glu-Glu-Ile-Pro-Glu-Glu-Phe(SO₃H)-Leu-OH,

Sar-Glu-Tyr-Glu-Glu-Ile-Pro-Glu-Glu-Phe(SO₃H)-Ile-OH,

Ac-Asn-Ala-Asp-Pgl-Glu-Glu-Ile-Pro-Glu-Glu-Phe(SO₃H)-Leu-OH,

H-Asp-Trp-Glu-Glu-Ile-Pro-Glu-Glu-Phe(SO₃H)-Leu-Gln-OH,

H-Asn-Gly-Asp-Pgl-Glu-Glu-Ile-Pro-Glu-Glu-Phe(SO₃H)-Leu-OH,

H-Asp-Asp-Phe-Glu-Glu-Ile-Pro-Glu-Glu-Phe(SO₃H)-Asp-OH,

Suc-Asp-Phe-Glu-Glu-Ile-Pro-Glu-Glu-Phe(SO₃H)-Leu-OH,

Suc-Asp-Pgl-Glu-Glu-Ile-Pro-Glu-Glu-Phe(SO₃H)-Leu-OH,

```
Ac-Gly-Asp-Phe-Glu-Glu-Nle-Pro-Glu-Glu-Phe(SO₃H)-
Ile-Asn-NH₂,

Ac-Gly-Asp-Tyr-Glu-Glu-Val-Pro-Glu-Glu-Phe(SO₃H)-
LeuNH₂,

Suc-Glu-Ala-Asp-Tyr-Glu-Pro-Leu-Pro-Glu-Glu-
Phe(SO₃H)-Leu-OH,

Ac-Gln-Ala-Asp-Phe-Asp-Asp-Phe-Asp-Asp-Phe(SO₃H)-Ala-
NH₂,

Ac-Gly-Asp-Phe-Glu-Glu-Ile-Pro-Glu-Glu-Phe(SO₃H)-
D-Leu-OH,

Ac-Gly-Asp-Phe-Glu-Glu-Ile-Pro-Glu-Glu-D-Phe(SO₃H)-
Leu-Gln-OH,

H-Asn-Gly-Asp-Phe-Glu-Glu-Ile-Pro-Glu-Glu-Pgl(SO₃H)-
Leu-OH,

Ac-Asp-Pgl-Glu-Glu-Ile-Pro-Glu-Glu-Pgl(SO₃H)-Leu-OH
oder

Ac-Asp-Nal-Glu-Glu-Ile-Pro-Glu-Glu-Phe(SO₃H)-Leu-OH.
```

**7.** Procédé pour la préparation d'un peptide selon la revendication 1, par synthèse de peptide en phase solide ou synthèse effectuée en solution.

**8.** Procédé pour la préparation d'un peptide selon la revendication 1, caractérisé en ce que la chaîne peptidique est synthétisée sur un support polymère, par couplage répétitif d'oligopeptides ou d'aminoacides protégés, et est ensuite séparée de celui-ci.

**9.** Procédé pour la préparation d'un peptide selon la revendication 1, caractérisé en ce que la chaîne peptidique est assemblée en solution, avec utilisation d'aminoacides protégés ou d'oligopeptides protégés, et le peptide est obtenu par élimination des groupes protecteurs.

**10.** Procédé pour la préparation d'un peptide de formule I, caractérisé en ce que des dérivés d'aminoacides protégés ou des segments peptidiques protégés sont assemblés les uns avec les autres, en solution ou sur une phase solide, et obtenus par élimination des groupes protecteurs ainsi que, dans le cas d'une synthèse sur phase solide, par séparation d'avec la résine de support, une cyclisation par oxydation pouvant être effectuée dans le cas de peptides contenant de la cystéine.

**EP 0 443 598 B1**

**11.** Produit diagnostique ou thérapeutique contenant un composé selon la revendication 1.

**Revendications pour les Etats contractants suivants : ES, GR**

**1.** Procédé pour la préparation d'un peptide de formule I

A1-A2-A3-A4-A5-A6-A7-A8-A9-A10-A11-A12-A13-A14-A15

dans laquelle

A1     est un atome d'hydrogène ou un reste cystéine, acétyl-cystéine, un ou deux groupes alkyle ayant de 1 à 4 atomes de carbone, un groupe acyle ayant de 2 à 10 atomes de carbone et un autre groupe carboxy, ou l'un des groupes protecteurs usuels dans la chimie des peptides,

A2     est une liaison, Asn, Asp, Gln ou Glu,

A3     est une liaison, Gly ou Ala,

A4     est Glu ou Asp,

A5     est Phe, Tyr, Trp, Pgl (phénylglycine) ou Nal (naphtylalanine),

A6     est Glu ou Asp,

A7     est Glu, Asp, Pro ou Ala,

A8     est Ile, Leu, Val, Nle ou Phe,

A9     est Pro ou Hyp,

A10    est Glu ou Asp,

A11    est Glu ou Asp,

A12    est $Phe(SO_3H)$ ou $Phe(PO_3H_2)$ (de préférence en position para) ou $Pgl(SO_3H)$ ou $Pgl(PO_3H_2)$ (de préférence en position para),

A13    est une liaison, Leu, Ile, Val ou Ala,

A14    est une liaison, Gln, Asn, Glu, Asp ou Cys et

A15    est Cys, Cys-amide, un groupe OH du groupe $\alpha$-carboxy, libre ou estérifié par un alcool inférieur ayant jusqu'à 4 atomes de carbone, qui peut également se trouver sous forme de fonction carboxamide, dont les atomes d'hydrogène peuvent éventuellement être remplacés par des groupes alkyle ayant jusqu'à 4 atomes de carbone,

par synthèse en phase solide ou synthèse effectuée en solution.

**2.** Procédé selon la revendication 1, caractérisé en ce que la chaîne peptidique est synthétisée sur un support polymère, par couplage répété d'aminoacides ou d'oligopeptides protégés, puis séparée de celui-ci.

**3.** Procédé selon la revendication 1, caractérisé en ce que la chaîne peptidique est assemblée en solution, avec utilisation d'aminoacides protégés ou d'oligopeptides protégés, et le peptide est obtenu par élimination des groupes protecteurs.

**4.** Procédé selon la revendication 1, caractérisé en ce que des dérivés d'aminoacides protégés ou des segments peptidiques protégés sont assemblés les uns avec les autres, en solution ou sur une phase solide, et obtenus par élimination des groupes protecteurs ainsi que, dans le cas d'une synthèse phase solide, par séparation d'avec la résine de support, une cyclisation par oxydation pouvant être effectuée dans le cas de peptides contenant de la cystéine.

**5.** Procédé selon l'une des revendications 1 à 4, caractérisé en ce que l'on prépare un peptide dans lequel A12 est un reste sulfophénylalanine sous la forme D ou L.

**6.** Procédé selon l'une des revendications 1 à 4, caractérisé en ce que l'on prépare un peptide dans lequel A12 est un reste sulfophénylglycine sous la forme D ou L.

**7.** Procédé selon l'une des revendications 1 à 4, caractérisé en ce que l'on prépare un peptide dans lequel A1 est un atome d'hydrogène ou le groupe méthyle, acétyle, benzoyle ou succinyle.

**8.** Procédé selon l'une des revendications 1 à 4, caractérisé en ce que l'on prépare un peptide ayant la structure

H-Asn-Gly-Asp-Phe-Glu-Glu-Pro-Glu-Glu-Phe($SO_3H$)-Leu-OH.

21

9. Procédé selon l'une des revendications 1 à 4, caractérisé en ce que l'on prépare un peptide ayant la structure

H-Asn-Gly-Asp-Phe-Glu-Glu-Ile-Pro-Glu-Glu-Phe(SO$_3$-H)-Leu-OH,

H-Gly-Asp-Phe-Glu-Glu-Ile-Pro-Glu-Glu-Phe(SO$_3$H)-Leu-Gln-OH,

Ac-Asp-Phe-Glu-Glu-Ile-Pro-Glu-Glu-Phe(SO$_3$H)-Leu--OH,

Sar-Glu-Tyr-Glu-Glu-Ile-Pro-Glu-Glu-Phe(SO$_3$H)-Ile--OH,

Ac-Asn-Ala-Asp-Pgl-Glu-Glu-Ile-Pro-Glu-Glu-Phe(SO$_3$H)-Leu-OH,

H-Asp-Trp-Glu-Glu-Ile-Pro-Glu-Glu-Phe(SO$_3$H)-Leu-Gln-OH,

H-Asn-Gly-Asp-Pgl-Glu-Glu-Ile-Pro-Glu-Glu-Phe(SO$_3$-H)-Leu-OH,

H-Asp-Asp-Phe-Glu-Glu-Ile-Pro-Glu-Glu-Phe(SO$_3$H)-Asp-OH,

Suc-Asp-Phe-Glu-Glu-Ile-Pro-Glu-Glu-Phe-(SO$_3$H)-Leu-OH,

Suc-Asp-Pgl-Glu-Glu-Ile-Pro-Glu-Glu-Phe(SO$_3$H)-Leu-OH,

Ac-Gly-Asp-Phe-Glu-Glu-Nle-Pro-Glu-Glu-Phe(SO$_3$H)-Ile-Asn-NH$_2$,

Ac-Gly-Asp-Tyr-Glu-Glu-Val-Pro-Glu-Glu-Phe(SO$_3$H)-LeuNH$_2$,

Suc-Glu-Ala-Asp-Tyr-Glu-Pro-Leu-Pro-Glu-Glu-Phe(SO$_3$H)-Leu-OH,

Ac-Gln-Ala-Asp-Phe-Asp-Asp-Phe-Asp-Asp-Phe(SO$_3$H)--Ala-NH$_2$,

Ac-Gly-Asp-Phe-Glu-Glu-Ile-Pro-Glu-Glu-Phe(SO$_3$H)-D-Leu-OH,

Ac-Gly-Asp-Phe-Glu-Glu-Ile-Pro-Glu-Glu-D-Phe(SO$_3$H-)-Leu-Gln-OH,

H-Asn-Gly-Asp-Phe-Glu-Glu-Ile-Pro-Glu-Glu-Pgl(SO$_3$-H)-Leu-OH,

Ac-Asp-Pgl-Glu-Glu-Ile-Pro-Glu-Glu-Pgl(SO$_3$H)-Leu--OH

ou

Ac-Asp-Nal-Glu-Glu-Ile-Pro-Glu-Glu-Phe(SO$_3$H)-Leu--OH.